(19) Europäisches Patentamt
European Patent Office
Office européen des brevets

(11) **EP 1 491 151 B1**

(12) **EUROPEAN PATENT SPECIFICATION**

(45) Date of publication and mention
of the grant of the patent:
**07.11.2007 Bulletin 2007/45**

(51) Int Cl.:
***A61B 17/17*** *(2006.01)* ***A61B 19/00*** *(2006.01)*

(21) Application number: **04447153.0**

(22) Date of filing: **28.06.2004**

(54) **Process for the acquisition of information intended for the insertion of a locking screw into an orifice of an endomedullary device**

Verfahren zur Erfassung Informationen um eine Schraube in einem Loch eines metallischen Gegenstands zu verriegeln

Procédé d'acquisition d'informations destinées à l'insertion d'une vis de verrouillage dans un orifice d'un objet métallique

(84) Designated Contracting States:
**AT BE BG CH CY CZ DE DK EE ES FI FR GB GR HU IE IT LI LU MC NL PL PT RO SE SI SK TR**

(30) Priority: **27.06.2003 EP 03447172**

(43) Date of publication of application:
**29.12.2004 Bulletin 2004/53**

(73) Proprietor: **Université Libre de Bruxelles**
**1050 Brussels (BE)**

(72) Inventors:
• **Leloup, Thierry**
**1160 Bruxelles (BE)**
• **Schuind, Frédéric**
**3220 Holsbeek (BE)**
• **Warzee, Nadine**
**1310 La Hulpe (BE)**

(74) Representative: **Van Malderen, Joëlle et al**
**pronovem - Office Van Malderen**
**Avenue Josse Goffin 158**
**1082 Bruxelles (BE)**

(56) References cited:
**DE-A- 10 037 491**     **US-A- 4 899 318**
**US-A1- 2003 088 179**     **US-B1- 6 285 902**

• **HOFSTETTER R ET AL: "COMPUTER-ASSISTED FLUOROSCOPY-BASED REDUCTION OF FEMORAL FRACTURES AND ANTETORSION CORRECTION" COMPUTER AIDED SURGERY, XX, XX, vol. 5, 2000, pages 311-325, XP001001432**

## Description

## Subject of the invention

The present invention relates to a process for the acquisition of information intended for the insertion of a locking screw into a distal orifice of an endomedullary device.

## Prior art

Closed fractures of the diaphysis of the femur and tibia are usually treated by intramedullary nailing. The principal difficulty relating to the intramedullary nailing technique lies in the fitting of the distal locking screws, which may prove very difficult as the holes for the nail are not directly visible by the surgeon. The proximal screws are generally inserted with the aid of a mechanical guide fixed to the nailing handle. Unfortunately, this process cannot be used for fitting the distal screws since the nail may twist and bend substantially while it is being inserted in order to match the shape of the intramedullary canal. The use of many fluoroscopic images is therefore inevitable. Several techniques have been developed to solve this problem.

The conventional technique consists in positioning the axis of the radioscopy unit perpendicular to the locking holes that thus appear perfectly circular in the images. Targeting is achieved using a drill or a Steinmann nail passed through a universal hands-free sight, or else using other mechanical assistance systems. An incision is made in the skin, level with the locking hole. The tip of the tool is placed against the external cortical wall, at the centre of the hole, and its axis is aligned with that of the image intensifier. This is performed under front and side fluoroscopic control. When the position and the orientation of the tool are satisfactory, the cortical walls may be drilled; the screw is then inserted. When using the fluoroscopic sight, the surgeon's access to the operating site is often restricted because of the presence of the radioscopic unit.

Several other devices have been developed for distal locking: mechanical targeting guides mounted on the proximal part of the nail; magnetic systems for locating the centre of the locking hole, etc. A number of patents relating to these techniques have been filed (e.g. US-B-5584838, US-A-2002058948, EP-A-1099413, US-B-6200316, US-B-6093192, US-B-5951561, US-B-5411503 and US-B-4541424). However, none solves the problem satisfactorily.

The technique of virtual fluoroscopy may also be used. This makes it possible to create the impression of continuous fluoroscopy in a certain direction on the basis of a single image. This method requires the position of the radioscopic unit and the surgeon's tools to be tracked using a three-dimensional locator and requires the projection of these tools to be superposed on the image initially acquired in real time. Several views corresponding to different orientations may be used simultaneously. Since the fluoroscopic images are distorted, a correction is needed: this is generally based on a grid of metal crosses or balls. For distal locking, two orthogonal views are acquired, one parallel and the other perpendicular to the axis of each hole. This requires the position of the radioscopic unit to be adjusted until the holes appear perfectly circular. The image thus obtained may be used for the virtual fluoroscopy that guides the insertion of the distal screws.

The technique of virtual fluoroscopy is used inter alia in US-B-6285902 and WO 03/043485A. The latter document discloses an improvement of the process described in the first document. The documents describe a method that allows distal locking of intramedullary nails (IN) on the basis of two fluoroscopic views and a graphical view representing a cross section of the nail at the locking hole. The latter view is generated from a 3D model of the IN obtained before the operation on the basis of data for manufacturing the implant. The model is produced using a conic projection model with three projection parameters in order to simulate the projection of the points in space on 2D images. Projection cones are therefore not constructed. Thanks to this 3D model, the orientation and the position of the distal holes are known relative to the proximal end of the nail. A mechanical device is used to fix light-emitting diodes on this end in a unique manner and the position of which relative to the proximal end of the IN is known by construction of the device. It follows that the position of the locking holes of the intramedullary nail is known relative to the position of the light-emitting diodes fixed to the proximal end of the nail. The method described in US-B-6285902 therefore considers the IN as a rigid element and takes absolutely no account of the bending and twisting that the IN may undergo while it is being inserted. In WO 03/043485A, bending of the nail is taken into account by applying a translational movement to the 3D model of the nail. The authors define the "centre of the nail" as the middle of the segment joining the centres of the two distal locking holes. The translational movement is calculated so as to bring into coincidence, on each image, the projections of the centre of the nail of the 3D model with the corresponding points located on each image. However, again no account is taken of the twisting of the nail.

Certain systems use tomodensitometric images to facilitate insertion of the nail and reduction of the fracture, but the distal locking also uses two views that must be taken from the front and from the side. A passive adjustable drilling guide is fixed to the proximal end of the nail. It is composed of a radiotransparent head and two concentric metal rings into which the drill is inserted. Its position is adjusted, as previously, with the aid of the image intensifier in order for the axis of the drill to coincide with that of the locking hole.

A final alternative is the CAOS system which uses a braked, passive articulated arm provided with a drilling guide in order to maintain alignment of the drilling motor. This is because the drill may skid upon contact with the cortical bone, the axis of the tool thus deviating from that of the locking hole. The guiding is based on a fluoroscopic view taken facing the locking holes: the surgeon must manipulate the articulated arm in order to centre the axis of the drilling guide with that of the hole. However, several adjustment images must be taken in order for the radioscopic unit to be precisely positioned.

Document US-B-4899318 discloses a method for retrieving a precise shape of the object on the basis of several fluoroscopic views by examining the transmission of the X-rays. The method is not applicable to the problem solved by the present application as the nail appears opaque and the transmission information is therefore unknown.

Document WO02/09611 discloses a technique for constructing an approximate model of an object (i.e. the proximal part of a femur) from two fluoroscopic views. The technique used is based on calculating the intersection of the conic projection surfaces of the object relative to the two views. This approximate model can be refined by taking additional images, in other directions, or by taking into account preoperative data coming from an MRI examination.

## Object of the invention

The object of the present invention is to provide an apparatus and a process for the acquisition of information intended for the insertion of a locking screw into a distal hole of an endomedullary device, and to do so with the aid of a guiding system based only on two images.

## Summary of the invention

The present invention relates to a process for the acquisition of information intended for the insertion of a locking screw into a distal locking hole of an endomedullary device, comprising the following steps:

- taking of two images of different orientations of the distal part of the endomedullary device using a radioscopic unit;
- acquisition of the projection parameters, especially the position of the X-ray source and of the projection plane, of each image by locating a reference frame fixed on the endomedullary device and optionally another reference frame fixed on the radioscopic unit;
- correction of any distortion of the images;
- segmentation of the distal part of the endomedullary device in each image and calculation of the attributes relating to the position of the device and to that of the holes, said attributes comprising at least the contours of said device, its centre of gravity and its principal axis;
- construction of the projection cone of the distal part of the device for each image;

- determination of the intersection of the two projection cones;
- modelling of the endomedullary device on the basis of the intersection obtained in the preceding step;
- determination of the centre of the locking hole with the aid of the modelling obtained in the preceding step and of the centres of gravity of the holes determined on the images;
- determination of the orientation of the locking orifice in an iterative manner; and
- guiding of the drilling tool.

In a preferred embodiment of the invention, this endomedullary device is an intramedullary nail.

According to another preferred embodiment, the images are fluoroscopic images.

Advantageously, the projection parameters for each image are acquired using a 3D optical locating system or a grid fixed directly to the said endomedullary device.

Preferably, a grid of radio-opaque features (for example, metal balls) is used so as to correct the distortions of the images and to automatically calculate the position of the irradiation source employed.

According to an advantageous embodiment, the intersection is calculated with the aid of a set of parallel planes perpendicular to the said principal axis of the device.

Typically, the modelling of the endomedullary device defines both the inner surface and the outer surface of the latter.

Preferably, the centre of each hole is determined on the basis of the position of this centre in each image.

According to a specific embodiment, the orientation of the locking hole is determined by envisaging all possible positions of the axis of the hole and by selecting that position which provides the highest degree of correspondence between a contour associated with the position of the axis and the contour of the hole present in said images.

According to an alternative embodiment, the method also includes the step of modelling a longitudinal slot in the endomedullary device.

Advantageously, the slot is modelled at least at the two holes by a rectangular parallelepiped.

Preferably, the process is performed using a drilling tool calibrated relative to a fixed reference frame on this tool or else using an adjustable mechanical drilling guide.

In a further object the invention relates to a program, executable on a programmable device, containing instructions, which, when executed, perform the method as described previously.

## Brief description of the drawings

Figure 1 shows a fluoroscopic image of the grid of radio-opaque features (metal balls) and of the distal end of an intramedullary nail.

Figure 2 shows attributes calculated during the segmentation.

Figure 3 shows a projection cone and various elements defined on the basis of the latter.

Figure 4 illustrates the fixing of a grid in the form of a frame directly on the nail.

Figure 5 illustrates the method of calculating the intersection of the two projection cones.

Figure 6 shows a set of closed contours (polygons) constituting the intersection of the projecting cones of an intramedullary nail.

Figure 7 shows the inscribed circle for a polygon contour of Figure 6.

Figure 8 shows the model of a nail consisting of an inner surface and an outer surface.

Figure 9 shows the orientation of the axes of the holes to be determined.

Figure 10 shows, for each hole, the four contours formed by the intersection of the two cylinders that model the nail and of the cylinder that models the envisaged hole.

Figure 11 shows the overall degree of correspondence as a function of the orientation of the axis of a hole.

Figure 12 shows the modelling of the slot.

Figure 13 shows four views for guiding the tool when aligning it with the axis of the orifice.

Figure 14 shows the errors in the orientation and positioning of the axes of the two holes during trials on dry bone.

Figure 15 shows the three points in question for calibrating the drilling tool.

## Detailed description of the invention

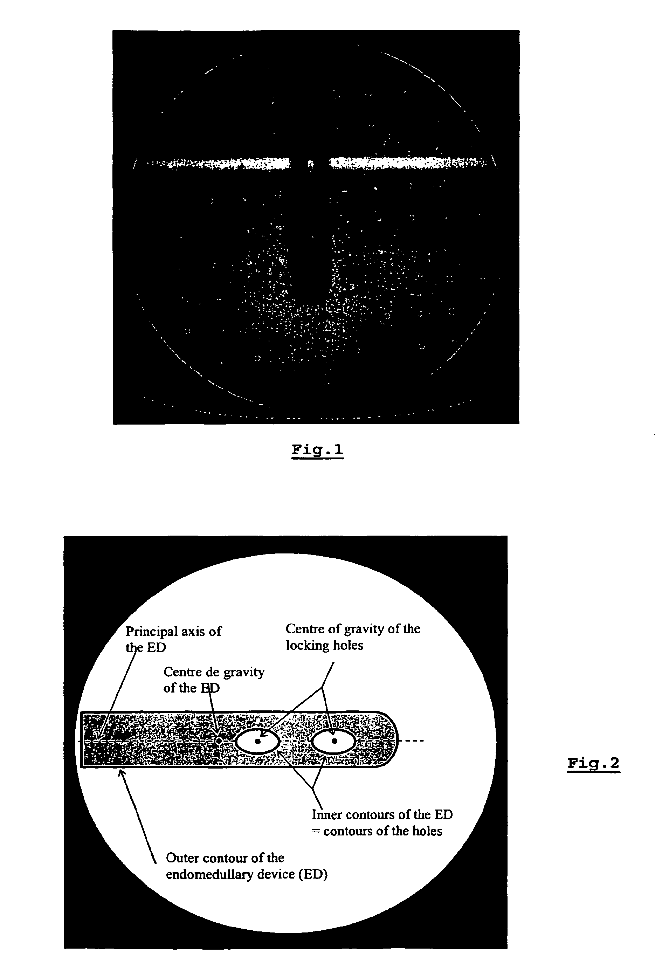

The information acquisition system disclosed in this invention requires the taking of two fluoroscopic images in which the holes for inserting the distal screws have to be visible. These two images therefore do not necessarily have to be taken strictly from the front and from the side (the holes may appear oval in the images). The projection parameters (three-dimensional positions of the X-ray source and of the image plane) of each view must be known in a reference frame rigidly fixed to the endomedullary device (for the detailed description of the invention, an intramedullary nail will be considered). One possible method consists in using a 3D optical locating system. Two reference frames (rigid bodies) are rigidly fixed to the nail and to a grid of metal balls that is placed on the image intensifier of the radioscopic unit. This grid is intended to correct the distortions of the fluoroscopic images and may include a second stage for automatically calculating the position of the source. Its central part is devoid of balls so as not to obscure the distal locking holes (Figure 1). The position of the grid of balls is known in the reference frame which is fixed thereto. The distortions of the two images are corrected using a conventional method described in the literature (modelling of the distortions by a two-dimensional polynomial constructed thanks to knowing the positions of the metal balls in reality and in the image).

The distal part of the intramedullary nail is segmented in each image by known image analysis techniques. The coordinates of the pixels constituting its contours (both external and internal) are recorded. Other attributes are also calculated, namely the principal axis of the object, its centroid, and those of the holes of the nail (Figure 2).

The linear transformation L that delivers, for each pixel, the corresponding three-dimensional point, expressed in the reference frame fixed to the intramedullary nail, is easily determined from knowledge of the three-dimensional coordinates of the points belonging to the grid and from their respective projection on the image in question (by minimizing a function of the sum of squares of distances).

The next step consists in constructing, for each view, the projection cone of the distal part of the intramedullary nail, expressed in the reference frame fixed to the patient.

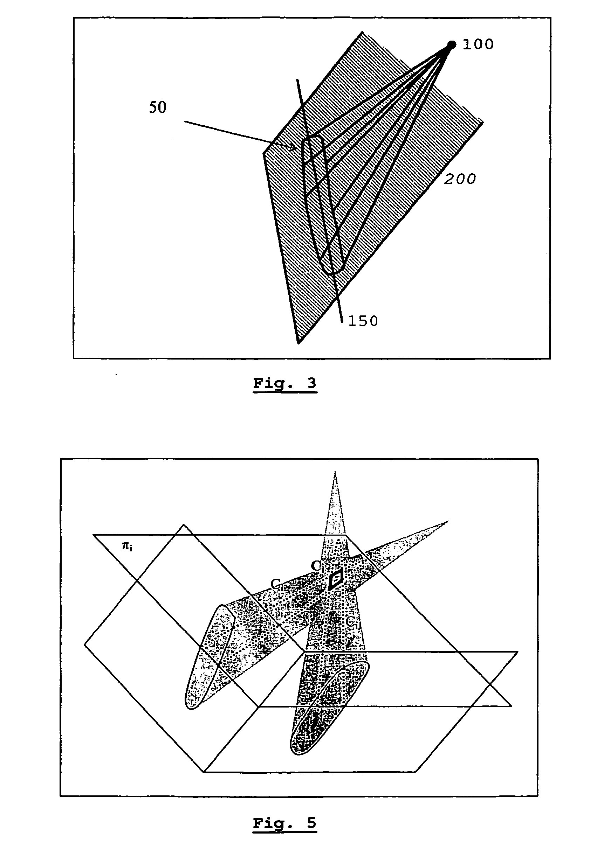

The transformation L is used to convert the two-dimensional external contour of the nail defined in the fluoroscopic image into a three-dimensional contour included in the plane of projection of the view in question. By joining each point of the contour (50) to the X-ray source, the projection cone of the intramedullary nail for the view in question, expressed in the reference frame fixed to the nail, is obtained. For each cone, a principal plane (200) may be defined (Figure 3): it contains the principal axis (150) of the projection of the object and it includes the X-ray source (100).

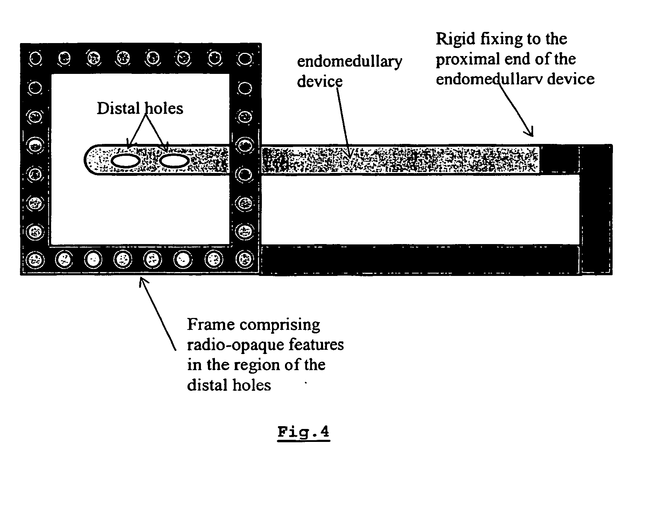

An alternative method consists in dispensing with the 3D locator and in fixing a grid directly on the nail (at the proximal end) so that it is placed in the region of the locking holes. This grid is in the form of a frame, so as to allow the locking to take place, while leaving it fixed to the nail (Figure 4). The grid also allows the image to be corrected and the projection parameters of each view to be obtained. These parameters are thus known directly in the reference frame fixed to the intramedullary nail.

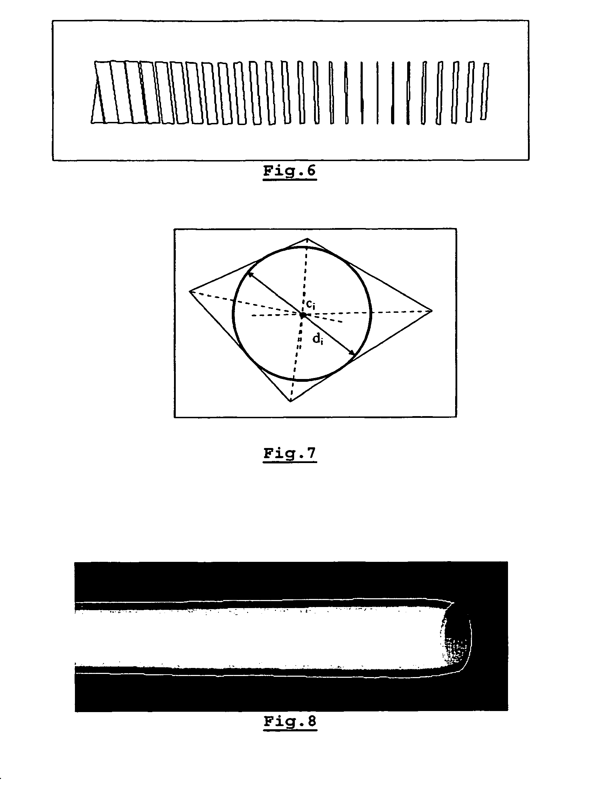

The construction of the three-dimensional model of the nail consists firstly in calculating the intersection of the two projection cones. This is performed by considering a set of parallel planes $\pi_i$, equally spaced and perpendicular to the principal axis of the nail. The intersection of the principal planes of the projection cones provides a good approximation of this axis. Each plane $\pi_i$ cuts the first projection cone along a closed contour $C_{i1}$. The same applies to the second cone: a contour $C_{i2}$ is defined (Figure 5). Both are included in the same plane, which makes it possible to calculate their intersection:

$$C_{i1} \cap C_{i2} = C_i.$$

By proceeding in this manner for each plane $\pi_i$, a set E = {$C_i$ ; i = 0, ..., n} of closed contours representing the intersection of the two projection cones is obtained (Figure 6).

Secondly, the nail is modelled by a generalized cylinder: for each contour of the set E, the centre $c_i$ and the diameter $d_i$ of the circle inscribed in the polygon in question (Figure 7) are calculated. These circles are approximated by regular polygons on which a triangular mesh modelling the external surface of the nail rests. Since the thickness of the metal is not negligible, the inner surface of the nail is also modelled by a triangular mesh resting on circles of the same centres as those used previously, but the radius of which has been reduced by the thickness of the metal (Figure 8).

The centres of the locking holes of the intramedullary nail are defined as the points of intersection between the axis of the nail and the axes of the holes, the latter theoretically being located in the same plane. In the model of the nail, the centres of the distal holes are therefore located along the central line (the line joining the centres of the previously calculated circles). The projection of the centre of each hole on the fluoroscopic image lies exactly at the centre of the corresponding inner contour of the segmented nail in the image. Consequently, to locate the centres of the holes on the central line, all that is required is to calculate the centre of gravity of each inner contour of the nail in the image, to convert the coordinates of these points into three-dimensional coordinates (see above), to determine, for each of these points, the straight line joining them to the X-ray source and to calculate the points of the central line that are closest to each straight line. Thus, for each view, an estimate is made of the position of the centres of the locking holes. An average of the estimates obtained for each hole is made, so as to determine the precise coordinates of the centres and to take account of all the information available. However, a step prior to this averaging consists in distinguishing, for each view, the most distal hole from the other one. This is performed by examining the proximity of each centre with the distal end of the nail.

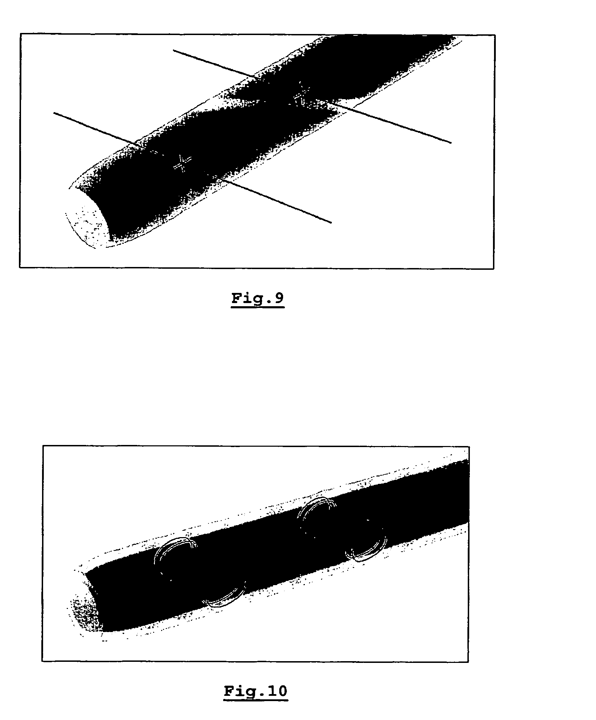

There are now generalized inner and outer cylinders of the nail, the central line of the latter and the centres of the two locking holes. The latter may be modelled by right cylinders, the axes of which each pass through the centre of the corresponding hole and are locally perpendicular to the central line. The diameter d of these cylinders may be measured using a sliding calliper. The sole missing data parameter is the orientation of the axes of the holes about the nail (Figure 9). Since the nail may undergo bending and twisting, it will be necessary to envisage the determination of each of the two axes. The method consists in envisaging all the possible positions of the axis in question, i.e. all the rotations of the axis about the central line, and in selecting that one which corresponds best to the images.

All that is required is to take account of a 180° rotation interval, since the cylinder is symmetrical. Thus, for each position in question of the axis of the hole, the degree of correspondence with the fluoroscopic images is calculated. This process requires several steps:

- firstly, the cylinder corresponding to the axis envisaged is constructed. It is formed from two circles (regular polygons) of diameters d (bases of the cylinder) and from generatrices parallel to its axis that connect corresponding points on the two circles, evenly spaced apart on the latter. The height h of the cylinder is chosen arbitrarily, but must be greater than the diameter of the nail;
- next, the intersections of this cylinder with the two generalized cylinders modelling the intramedullary nail are determined: there are in fact four contours resembling deformed circles (Figure 10). To calculate the intersection of a right cylinder modelling a hole with a generalized cylinder modelling the inner or outer surface of the nail, all that is required is to envisage each generatrix of the right cylinder and to search for its possible intersections with the triangles of the generalized cylinder that are considered separately. The problem is therefore reduced to calculating the intersection between a straight line and a triangle;
- the next step consists in projecting these contours onto the projection plane for each view. For this purpose, it is sufficient to project the points of the contours one by one using the inverse coordinate transformations of those described above. The intersection of these four two-dimensional contours forms a new contour corresponding to the hole that would be visible in the image if the orientation of its axis were that considered; and
- finally, this contour (called $C_E$) is compared with that of the corresponding hole, actually present in the image (which will be called $C_I$). Their intersection is determined and constitutes a contour $C_{EI}$. If the area of the inner surface of the closed contour C is denoted by A(C), it is possible to calculate a degree of correspondence between the contours $C_E$ and $C_I$ for the image i in question:

$$t_i = \frac{2A(C_{EI})}{A(C_E) + A(C_I)}.$$

The overall degree of correspondence is obtained by taking the average of the degrees of correspondence $t_i$ corresponding to the two fluoroscopic views:

$$t_G = \frac{1}{2}(t_1 + t_2).$$

Since the degree of correspondence of an image is always between 0 and 1, the same applies to the overall degree of correspondence.

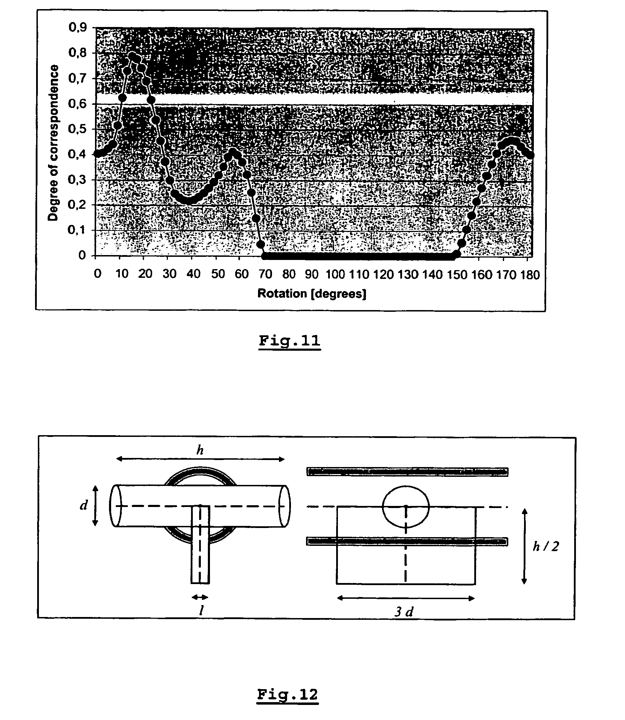

By repeating this process for each orientation in question of the axis and by recording the degree of correspondence obtained at each iteration, the graph of the overall degree of correspondence between the calculated hole and the actual hole may be represented as a function of the orientation of the former (Figure 11). All that is therefore necessary is to determine the highest overall degree of correspondence in order to determine the actual orientation of the axis of the locking hole in question. The same method is applied for determining the orientation of the axis of the second hole. The eight contours of intersection of the right cylinders modelling the locking holes with the inner and outer cylinders of the nail corresponding to the maximum overall degree of correspondence are recorded, so as to be able to displayed subsequently.

The method envisaged also makes it possible to model a longitudinal slot in the nail, and any deformation of this slot, for example as a result of a twist. It is relatively easy to introduce the longitudinal slot data into the model for the intramedullary nail. This is because, over a cross section of the nail, the direction of the slot is perpendicular to the axis of the locking holes. Since the nail may have been deformed when inserting it, it is modelled only over a short distance, separately for each hole. The modifications to be made to the process for seeking the orientation of the axis of a hole described in the previous paragraph are described below.

The slot corresponding to the hole in question is modelled by a rectangular parallelepiped which stops half way along the nail and does not pass right through it, like the right cylinders modelling the holes. One of the axes of this parallelepiped corresponds to the local direction of the nail, determined by the points on its central line in the vicinity of the centre of the hole in question. Its second axis corresponds to that of the hole. This determines the direction of the third axis, which is in fact the direction of the slot itself. One of the faces of this parallelepiped is centred on the centre of the hole, the opposed face, parallel to the first face, lies at a distance greater than the largest radius of the generalized cylinder modelling the outer surface of the nail, so as to ensure that the parallelepiped passes through the two generalized cylinders. Its width *l* depends on the width of the slot. The slot has to be modelled only in the vicinity of the hole in question, for example over a length of three times the diameter *d*. These measurements completely determine the size and the position of the parallelepiped (Figure 12).

As in the case of the right cylinders modelling the holes, its intersection with the generalized cylinders modelling the nail is then determined, which provides two additional contours per hole. The six contours are then grouped in pairs, namely the two contours corresponding to the slot, the two contours of the hole that are located on one side of the nail, and the two final contours located on the other side. These contours are then projected onto the plane of an image and the intersection of the contours belonging to the same pair is determined. This provides three contours $C_{O1}$, $C_{O2}$ and $C_F$, the latter corresponding to the intersection of the pair of contours coming from the slot of the nail. As it is impossible for an X-ray to pass simultaneously through the hole on either side and the slot in the nail, it is unnecessary to calculate the intersection of these three contours. However, the X-rays will not encounter metal in three situations:

- if they pass through the hole on either side;
- if they pass through the slot and one end of the hole; and
- if they pass through the slot and the other end of the hole.

This means that the intersections of the surfaces determined by the contours $C_{O1}$, $C_{O2}$ and $C_F$, taken in pairs, must be considered. The set E of these intersections represents, in the image in question, the regions internal to the contour of the nail where the X-rays do not encounter metal for the orientation in question of the hole axis. It therefore remains to compare E with the hole actually detected in the image, with a contour $C_I$, by calculating the intersection of E and $C_I$. By adopting the same notations as previously, the degree of correspondence of the orientation in question and of the actual orientation of the "hole+slot" combination is given by:

$$t_i = \frac{2A(E)}{A(E)+A(C_I)},$$

where $E = (C_{O1} \cap C_{O2}) \cup (C_{O1} \cap C_F) \cup (C_{O2} \cap C_F)$ and $A(E) = A(C_{O1} \cap C_{O2}) + A(C_{O1} \cap C_F) + A(C_{O2} \cap C_F)$. The mean of the $t_i$ values obtained for the two images gives the value of the overall degree of correspondence of the orientation in question with the actual situation. However, one should point out that, in this case, the orientation of the hole in question must be examined over a 360° interval since the "hole+slot" modelling combination is no longer symmetrical with respect to the axis of the nail.

Another object of the invention is to provide a guiding device for the insertion of the locking screws. In the case in which a 3D optical locating system is used, navigation software for displaying the three-dimensional model of the intramedullary nail may be used. The model may be displayed at any angle, but the most beneficial situations are those in which it is examined from a point of view lying on one of the axes of the holes ("guiding view"). In such a case, the four contours of the hole in question appear as concentric circles. A special function allows the nail to be displayed automatically in this manner for each hole and from each of its sides.

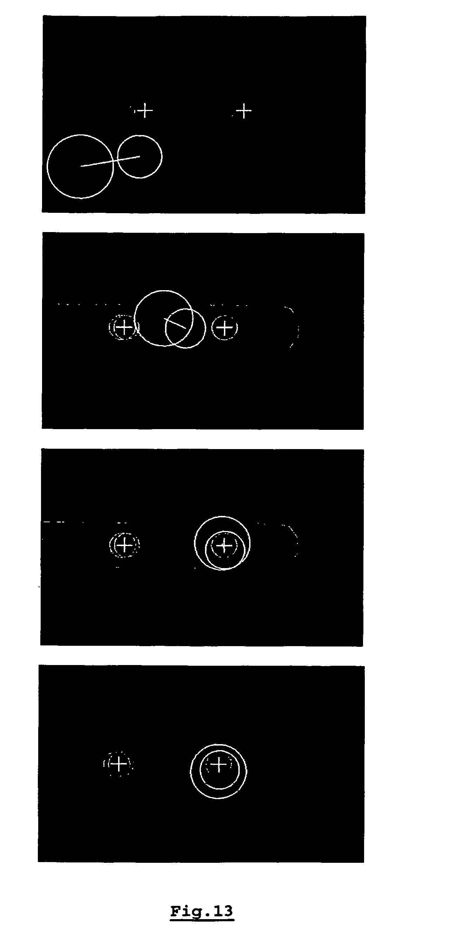

A reference frame is fixed to the drilling tool and a calibration process is used to precisely determine the position of the end of the drill bit and its orientation with respect to the tool's reference frame. During the guiding process of the surgical process, a straight line segment representing the bit of the tool is displayed in real time. Furthermore, two circles having the ends of this segment as centres and lying in planes perpendicular to this segment are also displayed so as to facilitate the alignment of the tool along the axis of the hole when the surgeon is using a guiding view. In fact, in such a case, he must ensure that the segment modelling the tool becomes a point centred on the hole in question, which means that it becomes almost invisible. The presence of these two circles allows another approach: to position his tool, the surgeon can disregard the segment modelling the bit of the tool and he can consider only the circles, the objective to be achieved being to make them both concentric and centred on the hole. In practice, both approaches are used: firstly, the surgeon positions his tool with reference to the segment and tries to reduce it to a point centred on the hole; secondly, when the segment becomes almost invisible, the use of the two circles proves to be much easier (Figure 13).

During guiding, the positions of the reference frame fixed to the nail and of that attached to the tool are digitized using the three-dimensional locator. The transformation of the coordinates of the second reference frame into the first is then determined. The ends of the segment modelling the tool are known in the reference frame of the latter. By applying the transformation calculated above to these two points, the coordinates of the ends of the segment in the reference frame of the nail are obtained, which allows the tool to be displayed on the three-dimensional image. This process is carried out iteratively. The nail therefore remains stationary, only the tool moving on the screen.

In the case in which only a grid fixed rigidly to the intramedullary nail is used, a mechanical guiding device that fits only onto the grid may be used. This device includes a drilling guide, that can be adjusted with four degrees of freedom (two in translation and two in rotation). Each degree of freedom is graduated on the device and can be locked. For each hole, the four corresponding values are calculated as a function of the known geometry and of the unique position of the device. These four degrees of freedom may be adjusted manually or by means of a robot. The drilling guide can thereafter be oriented precisely along the axis of the hole.

**Example 1 : Experimental trials on a single nail**

Several experimental trials of the navigation system were carried out. The experimental device adopted for each trial is described below.

A intramedullary nail was fixed to the operating table via its proximal part. The slot in the nail was oriented downwards, as during an actual operation (the patient being on his back, the concavity of the nail must be oriented downwards), the axes of the holes being approximately horizontal. The radiographic cassette holder was fixed to the image intensifier and the polycarbonate tray containing the balls of the grid was inserted thereinto. Two clamps were furthermore used to prevent any displacement of the grid due to a small gap existing between the latter and the holder. The radioscopic unit was installed, in order to allow the acquisition of views in which the distal locking holes of the nail appear in the region of the grid devoid of balls. A reference frame (a star tracked by the 3D locator) was fixed rigidly to the proximal part of the intramedullary nail. Instead of fixing another reference frame to the grid, four calibration points were defined and acquired using a pointer when the position of the grid has to be known. Since a single grid was used, the position of the X-ray source was also acquired using the pointer (digitization of two points located on either side of the source).

The six points intended for calculating the projection parameters of the radioscopic unit were acquired and the corresponding image was taken and then recorded. The second image was obtained in the same manner. These images were then corrected and the contours of the nail were determined in each view, allowing three-dimensional construction of the model of the nail. Since the fluoroscopy unit is then unnecessary, it is put to one side.

For the purpose of avoiding unnecessary desterilization of a drilling motor, the tool was modelled by a long locking screw. A reference frame was also fixed rigidly to the latter using an external fixer articulation. This screw had a diameter of 6.28 mm, while the bit normally used with the drill measured 5 mm in diameter. This allowed the screw provided with its reference frame to be locked in a locking hole of the nail, given the small clearance (0.22 mm), and thus allowed acquisition of the actual position and orientation of the hole. The calibration of the tool was performed by digitizing the head of the screw and its other end using the 3D pointer.

These various data were finally input into the navigation software, the desired three-dimensional view was selected and the guiding would be started. If the operator so wished, the corrected fluoroscopic images could be displayed alongside the three-dimensional view. The axis of the tool was projected onto these images and modified in real time as a function of its position.

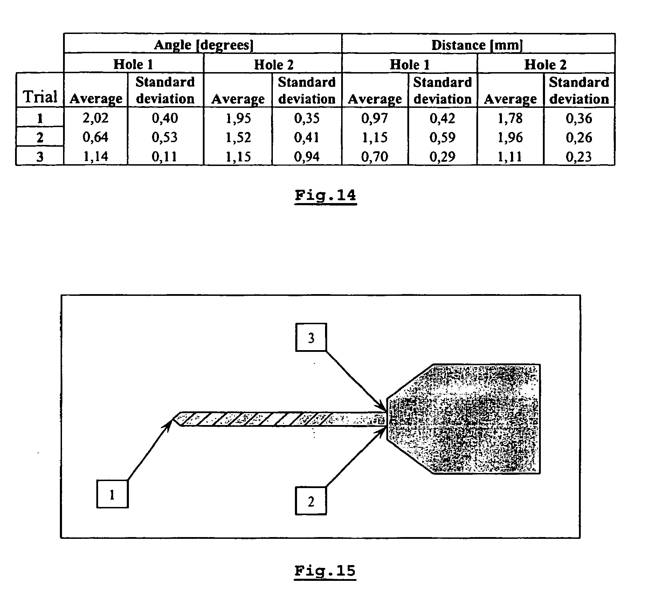

Three different trials were carried out, for which the position of the axis of the tool was recorded when it had been inserted into the locking holes. Since the clearance between the tool and the hole was very small, this axis provided a good measurement of the actual positions and orientation of the holes. To quantify the hole modelling error, the actual axes were compared with those calculated during our modelling. For each hole, the angle made between the actual axis and the calculated axis was calculated, as was the distance separating the actual axis from the calculated centre of the hole. Several positions of the tool were recorded for the same hole and the mean and standard deviations of the errors were calculated (Figure 14). This showed that the hole axis orientation error was around 2° and the positioning error was around 2 mm.

The calculation time needed to correct a fluoroscopic image did not exceed 30 seconds and that required for segmenting the nail in a corrected image was of the same order of magnitude. Construction of the three-dimensional model of the nail and of its holes took about 2 minutes. These times are acceptable for the operation in question.

**Example 2 : Experimental trials on a dry bone**

A plastic femur was sawn in two transversely, and the distal fragment was bored using a drill. The distal end of the nail was inserted into this fragment over a length of about 10 cm.

A universal drilling motor fitted with a 5 mm diameter bit was used to drill the hole for the locking screw. A reference frame was fixed to the handle of the tool by means of nylon clamps. A piece of rubber inserted between the two elements prevented them from slipping. The tool was calibrated by locating three points using the pointer (Figure 15), namely the end of the drill bit (1) and two diametrically opposed points located on the chuck of the tool (2-3). The axis of the drill bit was determined by the segment joining the first point to the mid-point between the two latter points. Apart from this, the experimental device was the same as that for the trials on just a nail.

It was found that, for 94% of the trials, the navigation system allowed the nail to be locked. In 70% of the cases, the drill bit passed directly through both holes of the orifice. One trial (in 17) failed since the operator moved the rigid body with respect to the tool when pressing on the latter. After recalibration of the tool, the drill bit was able to pass through the nail without any problems.

The images used by the system disclosed in this invention do not have to be taken from the front and from the side (the locking holes do not have to appear perfectly circular in one of the two images). The sole constraint lies in the visibility of the distal holes in each of the two images. Adjustment of the position of the radioscopic unit is thereby simplified, which reduces the irradiation of the patient and of the surgical team. In addition, the surgeon has available a three-dimensional view, which makes it possible to display simultaneously several views of the nail and of the tool at whatever angle; an extrapolation of the path of the drill bit may be displayed on the screen, and the error in the initial position may be calculated and supplied to the surgeon before insertion of the screw.

The process applies not only to cases with two or more parallel holes, but also to non-parallel holes. In the latter case, the same process applies using two images per hole.

The process of the invention is not limited to the example described above. The same process can be applied, for example, in the mechanical engineering sector, in which it is necessary in certain applications to insert a screw very accurately in difficult-to-reach places.

**Claims**

1. Process for the acquisition of information intended for the insertion of a locking screw into a distal locking hole of an endomedullary device, comprising the following steps:

   • taking of two images of different orientations of the distal part of the said endomedullary device using a radioscopic unit;
   • acquisition of the projection parameters especially the position of the X-ray source and of the projection plane, of each image by locating a reference frame fixed on the said endomedullary device and another reference frame fixed on said radioscopic unit;
   • correction of any distortion of the images;
   • segmentation of the distal part of the said endomedullary device in each image and calculation of the attributes relating to the position of the said device and to that of the holes, said attributes comprising at least the contours of the said device, its centre of gravity and its principal axis;
   • construction of the projection cone of the distal part of the device for each image;
   • determination of the intersection of the two projection cones;
   • modelling of the said endomedullary device on the basis of the said intersection obtained in the preceding step;
   • determination of the centre of the locking hole with the aid of the modelling obtained in the preceding step and of the centres of gravity of the holes determined on the images; and
   • determination of the orientation of the locking orifice in an iterative manner.

2. Process according to Claim 1, **characterized in that** the said endomedullary device is an intramedullary nail.

3. Process according to Claim 1 or 2, **characterized in that** the said images are fluoroscopic images.

4. Process according to any one of Claims 1 to 3, **characterized in that** the said projection parameters for each image are acquired using a 3D optical locating system or a grid fixed directly to the said endomedullary device.

5. Process according to any one of Claims 1 to 4, **characterized in that** a grid of radio-opaque features is used so as to correct the distortions of the images and to automatically calculate the position of the irradiation source employed.

6. Process according to any one of Claims 1 to 5, **characterized in that** the said intersection is calculated with the aid of a set of parallel planes perpendicular to the said principal axis of the said endomedullary device.

7. Process according to any one of Claims 1 to 6, **characterized in that** the said modelling of the said endomedullary device defines both the inner and outer surface of the endomedullary device.

8. Process according to any one of Claims 1 to 7, **characterized in that** the centre of each hole is determined on the basis of an estimate of the position of this centre in each image.

9. Process according to any one of Claims 1 to 8, **characterized in that** the orientation of the locking hole is determined by envisaging all possible positions of the axis of the hole and by selecting that position which provides the highest degree of correspondence between a contour associated with the position of the axis and the contour of the hole present in the said images.

10. Process according to any one of Claims 1 to 9, **characterized in that** the method also comprises a step of modelling a longitudinal slot in the said endomedullary device.

11. Process according to Claim 10, **characterized in that** the said slot is modelled at least at the two holes by a rectangular parallelepiped.

12. Process according to any one of Claims 1 to 11, **characterized in that** the process is carried out using a drilling tool calibrated with respect to a reference frame fixed to this tool or else using an adjustable mechanical drilling guide.

13. Computer program, executable by a programmable device, containing instructions which, when executed, perform the method as in any of the previous claims.

**Patentansprüche**

1. Verfahren für die Informationserfassung zwecks Einsetzens einer Verschlussschraube in ein distales Verschlussloch einer endomedullären Vorrichtung mit den folgenden Schritten:

   • Aufnahme zweier Bilder in verschiedenen Ausrichtungen des distalen Teils besagter endomedullärer Vorrichtung mithilfe einer radioskopischen Einheit;
   • Erfassen der Projektionsparameter, insbesondere der Position der Röntgenquelle, sowie der Projektionsebene, jeden Bildes durch Lokalisierung eines an besagter endomedullärer Vorrichtung angebrachten Referenzrahmens sowie eines weiteren, an besagter radioskopischer Einheit angebrachten Referenzrahmens;
   • Korrektur von Bildverzerrungen;
   • Segmentierung des distalen Teils besagter endomedullärer Vorrichtung in jedem Bild sowie Berechnung der Attribute mit Bezug auf die Position besagter Vorrichtung sowie die der Löcher; hierbei umfassen die besagten Attribute mindestens die Konturen der besagten Vorrichtung, ihren Schwerpunkt und ihre Hauptachsen;
   • Konstruktion des Projektionskonus des distalen Teils der Vorrichtung für jedes Bild;
   • Bestimmung der Schnittstelle der beiden Projektionskoni;
   • Modellierung der besagten endomedullären Vorrichtung auf der Grundlage besagter Schnittstelle, die durch den vorherigen Schritt erhalten wurde;
   • Mittelpunktbestimmung für das Verschlussloch mithilfe der im vorherigen Schritt erhaltenen Modellierung sowie der Schwerpunkte in Löchern wie in den Bildern festgestellt; sowie
   • Bestimmung der Ausrichtung der Verschlussmündung in einer iterativen Vorgehensweise.

2. Verfahren gemäß Anspruch 1, **dadurch gekennzeichnet, dass** es sich bei der besagten endomedullären Vorrichtung um einen intramedullären Nagel handelt.

3. Verfahren gemäß Anspruch 1 oder 2, **dadurch gekennzeichnet, dass** es sich bei besagten Bildern um fluoroskopische Bilder handelt

4. Verfahren gemäß einem der Ansprüche 1 bis 3, **dadurch gekennzeichnet, dass** die besagten Projektionsparameter für jedes Bild mithilfe eines optischen 3D-Lokalisierungssystems oder einem Gitter erfasst werden, das direkt an der endomedullären Vorrichtung befestigt ist.

**5.** Verfahren gemäß einem der Ansprüche 1 bis 4, **dadurch gekennzeichnet, dass** ein Gitter mit röntgendichten Markmale verwendet wird, um die Bildverzerrungen zu korrigieren und automatisch die Position der verwendeten Strahlungsquelle zu berechnen.

**6.** Verfahren gemäß einem der Ansprüche 1 bis 5, **dadurch gekennzeichnet, dass** die besagte Schnittstelle mithilfe einer Reihe paralleler Ebenen senkrecht zur besagten Hauptachse der besagten endomedullären Vorrichtung berechnet wird.

**7.** Verfahren gemäß einem der Ansprüche 1 bis 6, **dadurch gekennzeichnet, dass** die besagte Modellierung der besagten endomedullären Vorrichtung sowohl die innere als auch die äußere oberfläche der besagten endomedullären Vorrichtung definiert.

**8.** Verfahren gemäß einem der Ansprüche 1 bis 7, **dadurch gekennzeichnet, dass** der Mittelpunkt jedes Lochs auf der Grundlage einer Schätzung der Position dieses Mittelpunkts in jedem Bild bestimmt wird.

**9.** Verfahren gemäß einem der Ansprüche 1 bis 8, **dadurch gekennzeichnet, dass** die Ausrichtung des Verschlussloches bestimmt wird, indem sämtliche möglichen Positionen der Achse des Loches vorgesehen werden und die Position ausgewählt wird, die den größten Grad an Übereinstimmung zwischen einer mit der Position dieser Achse in Verbindung gebrachten Kontur sowie der Kontur des in den besagten Bildern vorhandenen Loches bietet.

**10.** Verfahren gemäß einem der Ansprüche 1 bis 9, **dadurch gekennzeichnet, dass** das Verfahren ferner einen Schritt zur Modellierung eines Längsschlitzes in der besagten endomedullären Vorrichtung umfasst.

**11.** Verfahren gemäß Anspruch 10, **dadurch gekennzeichnet, dass** der besagte Schlitz an mindestens zwei Löchern durch einen rechteckigen Parallelepiped modelliert wird.

**12.** Verfahren gemäß einem der Ansprüche 1 bis 11, **dadurch gekennzeichnet, dass** das Verfahren mithilfe eines Bohrwerkzeuges durchgeführt wird, das im Hinblick auf einen an diesem Werkzeug befestigten Referenzrahmen kalibriert wird oder andernfalls mithilfe einer einstellbaren mechanischen Bohrführung.

**13.** Computerprogramm, durch eine programmierbare Vorrichtung ausführbar, das Anweisungen enthält, die bei entsprechender Ausführung das Verfahren wie in den vorherigen Ansprüchen dargestellt durchführen.

**Revendications**

**1.** Procédé d'acquisition d'informations destinées à l'insertion d'une vis de verrouillage dans un orifice de verrouillage distal d'un dispositif endomédullaire, constitué des étapes suivantes :

• Prise de deux images à angles de vue différents de la partie distale dudit dispositif endomédullaire à l'aide d'un appareil de radioscopie,
• Acquisition des paramètres de projection, notamment la position de la source de rayons X et du plan de projection, de chaque image en localisant un repère fixé audit dispositif endomédullaire et un autre repère fixé audit appareil de radioscopie,
• Correction des distorsions des images,
• segmentation de la partie distale dudit dispositif endomédullaire sur chaque image et calcul des attributs relatifs à la projection dudit dispositif ainsi qu'à celle des orifices, lesdits attributs comprenant au moins les contours dudit dispositif, son centre de gravité et son axe principal,
• Construction du cône de projection de la partie distale du dispositif pour chaque image,
• Détermination de l'intersection des deux cônes de projection,
• Modélisation dudit dispositif endomédullaire à partir de ladite intersection obtenue dans l'étape précédente,
• Détermination du centre de l'orifice de verrouillage à l'aide de la modélisation obtenue dans l'étape précédente et des centres de gravité des orifices déterminés sur les images,
• Détermination de l'orientation de l'orifice de verrouillage d'une façon itérative.

**2.** Procédé selon la revendication 1, **caractérisé en ce que** ledit dispositif endomédullaire est un clou intramédullaire.

**3.** Procédé selon la revendication 1 ou 2, **caractérisé en ce que** lesdites images sont des images fluoroscopiques.

**4.** Procédé selon l'une quelconque des revendications 1 à 3, **caractérisé en ce que** lesdits paramètres de projection de chaque image sont acquis à l'aide d'un système optique de localisation 3D ou d'une grille fixée directement sur ledit dispositif endomédullaire.

**5.** Procédé selon l'une quelconque des revendications 1 à 4, **caractérisé en ce que** une grille de motifs radio-opaques est utilisée afin de corriger les distorsions des images et de calculer automatiquement la position de la source d'irradiation employée.

**6.** Procédé selon l'une quelconque des revendications 1 à 5, **caractérisé en ce que** le calcul de ladite intersection s'effectue à l'aide d'un ensemble de plans parallèles, perpendiculaires audit axe principal dudit dispositif endomédullaire.

7. Procédé selon l'une quelconque des revendications 1 à 6, **caractérisé en ce que** ladite modélisation dudit dispositif endomédullaire définit à la fois la surface intérieure et extérieure du dispositif endomédullaire.

8. Procédé selon l'une quelconque des revendications 1 à 7, **caractérisé en ce que** la détermination du centre de chaque orifice est réalisée à partir d'une estimation de la position de ce centre sur chaque image.

9. Procédé selon l'une quelconque des revendications 1 à 8, **caractérisé en ce que** l'orientation de l'orifice de verrouillage est déterminée en envisageant toutes les positions possibles de l'axe de l'orifice et en sélectionnant celle qui fournit le taux de correspondance le plus élevé entre un contour lié à la position de l'axe et le contour de l'orifice présent sur lesdites images.

10. Procédé selon l'une quelconque des revendications 1 à 9, **caractérisé en ce que** la méthode comprend également une étape de la modélisation d'une fente longitudinale dudit dispositif endomédullaire.

11. Procédé selon la revendication 10, **caractérisé en ce que** ladite fente est modélisée au moins au niveau des deux orifices par un parallélépipède rectangle.

12. Procédé selon l'une quelconque des revendications 1 à 11, **caractérisé en ce que** le procédé est réalisé à l'aide d'un l'outil de méchage calibré par rapport à un repère fixé à cet outil ou bien à l'aide d'un guide de méchage mécanique ajustable.

13. Programme informatique, exécutable par un dispositif programmable, contenant des instructions, qui, lorsqu'elles sont exécutées, mettent en oeuvre le procédé selon l'une quelconque des revendications précédentes.

**Fig.1**

Principal axis of the ED

Centre of gravity of the locking holes

Centre de gravity of the ED

Inner contours of the ED = contours of the holes

Outer contour of the endomedullary device (ED)

**Fig.2**

**Fig. 3**

**Fig. 5**

Distal holes

endomedullary device

Rigid fixing to the proximal end of the endomedullary device

Frame comprising radio-opaque features in the region of the distal holes

**Fig.4**

**Fig.6**

**Fig.7**

**Fig.8**

**Fig.9**

**Fig.10**

**Fig.11**

**Fig.12**

**Fig.13**

| Trial | Angle [degrees] | | | | Distance [mm] | | | |
|---|---|---|---|---|---|---|---|---|
| | Hole 1 | | Hole 2 | | Hole 1 | | Hole 2 | |
| | Average | Standard deviation | Average | Standard deviation | Average | Standard deviation | Average | Standard deviation |
| 1 | 2,02 | 0,40 | 1,95 | 0,35 | 0,97 | 0,42 | 1,78 | 0,36 |
| 2 | 0,64 | 0,53 | 1,52 | 0,41 | 1,15 | 0,59 | 1,96 | 0,26 |
| 3 | 1,14 | 0,11 | 1,15 | 0,94 | 0,70 | 0,29 | 1,11 | 0,23 |

Fig.14

Fig.15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5584838 B
- US 2002058948 A
- EP 1099413 A
- US 6200316 B
- US 6093192 B
- US 5951561 B
- US 5411503 B
- US 4541424 B
- US 6285902 B
- WO 03043485 A
- US 4899318 B
- WO 0209611 A